# EUROPÄISCHE PATENTANMELDUNG

(11) **EP 2 090 651 A1**
(43) Veröffentlichungstag der Anmeldung: **19.08.2009**
(21) Anmeldenummer: 08101693.3
(22) Anmeldetag: 15.02.2008
(51) Int. Cl.: C12N 9/24, C12N 15/56, C12P 19/26, C07H 15/00, C12N 15/63

(54) **Chitinosanase**

(71) Anmelder: Westfälische Wilhelms-Universität Münster, 48149 Münster (DE)
(72) Erfinder: Moerschbacher, Bruno, 48161 Münster (DE); El Gueddari, Nour Eddine, 48143 Münster (DE); Kohlhoff, Markus, Dr., 30161 Hannover (DE)
(74) Vertreter: Helbing, Jörg

(57) **Zusammenfassung**

Die vorliegende Erfindung betrifft ein als Chitinosanase bezeichnetes Chitosanabbauendes Enzym aus dem Pilz *Alternaria alternata*, das spezifisch die glykosidische Bindung GIcNAc-GIcN im Chitosan spaltet, DNA Sequenzen die dieses Enzym kodieren, Vektoren und Wirtszellen mit dieser DNA Sequenz, die Herstellung dieses Enzyms, sowie dessen Verwendung zur Spaltung von Chitosan.

## Beschreibung

Die vorliegende Erfindung betrifft ein als Chitinosanase bezeichnetes Chitosan-abbauendes Enzym aus dem Pilz *Alternaria alternata*, das spezifisch die glykosidische Bindung GlcNAc-GlcN im Chitosan spaltet, DNA Sequenzen die dieses Enzym kodieren, Vektoren und Wirtszellen mit dieser DNA Sequenz, die Herstellung dieses Enzyms, sowie dessen Verwendung zur Spaltung von Chitosan.

### Hintergrund der Erfindung

Chitosan ist ein lineares Co-Polymer aus Glukosamin (GlcN, D) und N-Acetyl-Glukosamin (GlcNAc, A). Chitosan wird kommerziell aus Chitin, einem vollständig acetylierten GlcNAc-Polymer hergestellt, entweder durch partielle De-N-Acetylierung oder durch vollständige De-N-Acetylierung und anschließende partielle Re-N-Acetylierung. In beiden Fällen erhält man aufgrund der chemischen Verfahrensweise partiell acetylierte Chitosane mit einer zufälligen Verteilung der Acetylreste entlang der linearen Grundkette.

| | | |
|---|---|---|
| Chitin | AAAAAAAAAAAAAAAAAAAAAAAAAAAAAAAAAAAAA | DA 100 % |
| PolyGlukosamin | DDDDDDDDDDDDDDDDDDDDDDDDDDDDDDDDDD | DA 0 % |
| Chitosan (z.B.) | DDDAADDDADAADAAADDAADDAAADDADDDADAAA | DA 50 % |

Die Polymere können entweder physikalisch, chemisch oder enzymatisch depolymerisiert werden. Die physikalische Depolymerisation erfolgt durch Ultraschallbehandlung, ihre Spezifität hinsichtlich der Frage, an welcher Stelle das Polymer fragmentiert wird, ist noch unbekannt. Die chemische Depolymerisation erfolgt normalerweise rein zufällig (zufällige saure Hydrolyse) oder, wenn unter genau definierten Bedingungen gearbeitet wird, hinter jedem "A" (partielle saure Hydrolyse) oder hinter jedem "D" (oxidative Deaminierung), wobei die D-Einheit zur Anhydro-Mannose (M) deaminiert wird. Für das obige Chitosan ergäbe sich:
zuf. saure Hydr. DDDA ADD DAD AADA AAD DA ADDA AA DDA D DDA DA AA
part. saure Hydr. DDDA A DDDA DA A DA A A DDA A DDA A A DDA DDDA DA A A
oxid. Deamin. M M M AAM M M AM AAM AAAM M AAM M AAAM M AM M M AM AAA

Die enzymatische Depolymerisation kann entweder mit Chitinasen (nachfolgend "Chitin.") oder mit Chitosanasen (nachfolgend "Chitos.") erfolgen. Alle Chitinasen spalten zwischen "AA", manche zusätzlich auch zwischen "AD" oder "DA". Alle Chitosanasen spalten zwischen "DD", manche zusätzlich auch zwischen "DA" oder "AD". Für das obige Chitosan ergäbe sich:

| | |
|---|---|
| Chitin. AA | DDDA ADDDADA ADA A ADDA ADDA A ADDADDDADA A A |
| Chitin. AA/AD | DDDA A DDDA DA A DA A A DDA A DDA A A DDA DDDA DA A A |
| Chitin. AA/DA | DDD A ADDD AD A AD A A ADD A ADD A A ADD ADDD AD A A A |
| Chitos. DD | D D DAAD D DADAADAAAD DAAD DAAAD DAD D DADAAA |
| Chitos. DD/DA | D D D AAD D D AD AAD AAAD D AAD D AAAD D AD D D AD AAA |
| Chitos. DD/AD | D D DAA D D DA DAA DAAA D DAA D DAAA D DA D D DA DAAA |

Die Bereitstellung einer Chitosanase mit solch einer definierten Substratspezifität wäre höchst wünschenswert.

### Kurzbeschreibung der Erfindung

Es wurde ein Chitosan-abbauendes Enzym aus dem Pilz *Alternaria alternata* isoliert und charakterisiert und festgestellt, dass dieses Enzym eine bisher nicht beschriebene Substratspezifität aufweist, die es eindeutig von den bisher bekannten Chitinasen und Chitosanasen unterscheidet. Das gefundene Enzym hat eine absolute Spezifität für die Spaltung von "AD". Es handelt sich demnach weder um eine typische Chitinase, die als gemeinsame Eigenschaft die Fähigkeit aufweisen, "AA" zu spalten, noch um eine typische Chitosanase, da diese alle "DD" spalten können. Das Enzym definiert also eine neue Klasse von Chitosan-hydrolysierenden Enzymen, die wir hier als "Chitinosanasen" (Chitinos.) bezeichnen. Als Abbauprodukte des obigen Chitosans ergäben sich:

| | |
|---|---|
| Chitinos. AD | DDDAA DDDA DAA DAAA DDAA DDAAA DDA DDDA DAAA |

Bereits dieses sehr begrenzte Beispiel zeigt: die verschiedenen Methoden ergeben recht unterschiedliche Produktmischungen. Tatsächlich ist die Vielfalt noch größer, da die Spezifitäten der Enzyme vereinfacht dargestellt sind. So wurde außer Acht gelassen, dass auch die Zuckereinheit neben der eigentlichen Spaltstelle entscheidend sein kann (so spaltet z.B. menschliches Lysozym "AAA" in "AA A"), das die Spezifitäten oft nicht absolut sondern nur partiell sind, und dass viele Enzyme eine Mindestoligomerlänge benötigen, um dieses als Substrat zu akzeptieren (so spalten die meisten Chitinasen "AAAA" in zwei Moleküle "AA" oder in je ein Molekül "A" und "AAA", die Produkte dieses Abbaus, also das Trimer "AAA" und das Dimer "AA", können aber nicht weiter gespalten werden). Auffallend ist, dass alle Produktmischungen, egal ob sie chemisch oder enzymatisch mit Chitinasen oder Chitosanasen hergestellt werden, durch eine hohe Komplexität des Produktgemischs gekennzeichnet sind. Um dies zu veranschaulichen und die Produkte der unterschiedlichen Methoden besser vergleichen zu können, sollen die oligomeren Produkte nach Größe sortiert werden:

| | |
|---|---|
| zuf. saure Hydrol.D | DA DA AA AA DAD ADD DDA DDA AAD DDDA ADDA AADA |
| part. saure Hydr. | A A A A A A A A A DA DA DA DDA DDA DDA DDDA DDDA DDDA |
| oxid. Deamin. AAAM | M M M M M M M M M M AM AM AM AAM AAM AAM AAA AAAM |
| Chitin. AA | A A A A ADA DDDA ADDA ADDA ADDDADA ADDADDDADA |
| Chitin. AA/AD | A A A A A A A A A DA DA DA DDA DDA DDA DDDA DDDA DDDA |
| Chitin. AA/DA | A A A A A A A A A A AD AD AD DDD ADD ADD ADD ADDD ADDD |
| Chitos. DD | D D D D DAD DAAD DAAD DAAAD DADAAA DADAADAAAD |
| Chitos. DD/DA | D D D D D D D D D D AD AD AD AAD AAD AAD AAA AAAD AAAD |
| Chitos. DD/AD | D D D D D D D D D DA DA DA DAA DAA DAA DAAA DAAA DAAA |
| Chitinos. AD | DDA DAA DDDAA DDDA DDDA DDAA DAAA DAAA DDAAA |

Es fällt auf, dass bei fast allen Verfahren sehr kleine Produkte auftreten und diese auch oft überwiegen. Eine Ausnahme bildet hier die Chitinosanase. Hinzu kommt, dass nur bei Verwendung der hochspezifischen Enzyme, die nur einen Bindungstyp, sei es "AA", "DD" oder "AD", spalten, auch größere Oligomere entstehen. Es scheint jedoch, dass für spezifische Interaktionen mit Proteinen (z.B. Enzymen, Rezeptoren, etc.) und damit für biologische Aktivitäten, eine Mindestgröße von vier, eher fünf Zuckerresten notwendig ist, so dass gerade größere Oligomere von biotechnologischem und biomedizinischem Interesse sind.

Betrachtet man die entstandenen Oligomere genauer, so fällt auf, dass sich die Produkte der partiellen sauren Hydrolyse ebenso wie die Produkte der Chitinase AA/AD mit der Formel DₙA beschreiben lassen, die der Chitinase AA/DA mit der Formel ADₙ, es handelt sich also in diesen drei Fällen um Glukosaminoligomere mit einer einzigen Acetylgruppe, die entweder am reduzierenden oder am nicht-reduzierenden Ende sitzt. Die Produkte der oxidativen Deaminierung entsprechen der Formel AₙM, die der Chitosanasen DD/DA und DD/AD den Formeln AₙD, und DAₙ, es handelt sich in diesen drei Fällen also um N-Acetyl-Glukosaminoligomere, denen eine Acetylgruppe entweder am reduzierenden oder am nicht-reduzierenden Ende fehlt. Die Produkte der zufälligen sauren Hydrolyse zeigen keinerlei Regelmäßigkeit, die der Chitinase AA und der Chitosanase DD lediglich das Vorhandensein von A- bzw. D-Resten an beiden Enden der Kette, auch in diesen beiden Fällen ist aber das Vorhandensein und die Verteilung von A- und D-Resten in der Mitte der Oligomere fast zufällig (außer dass bei den Produkten der Chitinase AA nur einzelne A-Reste und bei denen der Chitosanase DD nur einzelne D-Reste vorkommen).

Im Gegensatz dazu liefert die Chitinosanase partiell acetylierte Oligomere der Formel DₙAₘ, bei denen alle D-Reste am nicht-reduzierenden Ende, alle A-Rest am reduzierenden Ende als Block auftreten. Solche Oligomere lassen sich mit keinem bisher bekannten Verfahren möglicherweise mit Ausnahme einer sehr aufwendigen chemischen Synthese herstellen.

Die Erfindung betrifft somit
(1) eine Chitinosanase, die aus dem Pilz *Alternaria alternata* erhältlich ist und die:
   (a) spezifisch die glykosidische Bindung GlcNAc-GlcN (A-D) im Chitosan spaltet,
   (b) ein mittels SDS-PAGE bestimmtes relatives Molekulargewicht von etwa 18 kDa besitzt,
   (c) ein pH-Optimum bei pH 4 besitzt und
   (d) ein Temperatur-Optimum bei 70°C besitzt;
(2) eine Chitinosanase, insbesondere eine bevorzugte Ausführungsform von der Chitinosanase (1), die
   (a) mit einer Aminosäuresequenz, die eines oder beide der in SEQ ID NO: 1 und 2 gezeigten Proteinfragmente aufweist,
   (b) ein Sequenzhomolog von (a) ist, das eine Ähnlichkeit von wenigstens 80% zu der Sequenz (a) aufweist,
   (c) ein Fragment von (a) oder (b) ist, das wenigstens 10 zusammenhängende Aminosäurereste der Sequenz (a) oder (b) aufweist, oder
   (d) ein Derivat von (a), (b) oder (c) ist;
(3) eine DNA Sequenz, die eine Chitinosanase nach (1) oder (2) codiert;
(4) ein Vektor, der eine DNA Sequenz nach (3) enthält;
(5) eine Wirtszelle, die mit dem Vektor nach (5) transformiert/transfiziert ist und/oder die DNA Sequenz nach (3) aufweist;
(6) ein Verfahren zur Herstellung und einer Chitinosanase nach (1) oder (2), umfassend das Kultivieren einer Wirtszelle nach (5) und Isolieren der Chitinosanase aus den kultivierten Wirtszellen und/oder aus der Kulturlösung;
(7) eine Enzymzusammensetzung enthaltend eine Chitinosanase nach (1) oder (2);
(8) ein Verfahren zum Abbau von Chitosan, umfassend die Umsetzung des Chitosans mit einer Chitinosanase nach (1) oder (2), mit einer Wirtszelle nach (5) oder mit einer Enzymzusammensetzung nach (7) und Isolieren der Chitosanabbauprodukte; und
(9) Chitosanabbauprodukte erhältlich nach einem Verfahren nach (8).

### Kurzbeschreibung der Figuren

Fig. 1: Aufreinigung der Chitinosanase. Fig. 1a: FPLC-Chromatogramm (Säule: Mono S; Probe: konzentriertes Medium von *A. alternata* (0,1% w/v); Puffer: 50 mM Na-Acetat pH 4; Flussrate 0,25 mL/min; Elution: 0-1 M NaCl; Fraktionen: 1 mL);
Fig. 1b: Dot-assay der FPLC-Fraktionen (Substrat: Chitosan DA 64 %); Fig. 1c: SDS-PAGE (12%) der Fraktion 10 mit (A) Coomassie-Färbung und (B) Zymogramm (Substrat: Chitosan DA 64 %).
Fig. 2: pH-Optimum (A), Temperatur-Optimum (B) und Temperatur-Stabilität (C) der Chitinosanase. A: verwendete Puffer (jeweils 50 mM): pH 1,5-2,5: Glycin/Cl; pH 3,0-7,0: Citrat/Phosphat; pH 8,0-9,0. Tris/Cl; pH 9,0-11,0: Carbonat; Inkubation: 2 h bei 60°C; B: Inkubation: 2 h in 50 mM Na-Acetat pH 4,3; C: Lagerung bei 4°C (Quadrate), 37°C (Kreise), 60°C (Dreiecke) oder 80°C (Trapeze); Inkubation: 2 h bei 60°C in 50 mM Na-Acetat pH 4,3. In allen Experimenten wurde Chitosan DA 66 % als Substrat verwendet.
Fig. 3: Substratspezifität der Chitinosanase. Enzym: 4,5 pkat gereinigte Chitinosanase Substrat: 20 µg Chitosan des jeweiligen DA Inkubation: 15 h bei 37°C in 50 mM Na-Acetat pH 4,3.
Fig. 4: Massenspektren der Produkte des Chitinosanase-Abbaus von Chitosanen mit unterschiedlichem DA (%). Inkubation: 4.5 pkat gereinigte Chitinosanase; 15 h bei 37°C in 20 mM Na-Acetat pH 4.3; Analyse: MALDI-TOF-MS; Produkte sind mit DxAy bezeichnet; x = Anzahl GlcN, y: Anzahl GlcNAc.
Fig. 5: NMR Analyse der Chitinosanase-Produkte. Inkubation: 50 pkat gereinigte Chitinosanase; 24 h bei 37°C in 50 mM Citrat pH 4,3 Analyse: 400 MHz ¹H-NMR. Zum Vergleich wurde ein partiell Säure-hydrolysiertes Chitosan analysiert (oben).

### Sequenzprotokoll (freier Text)

### SEQ ID Nos:1 und 2 Chitinosanasefragmente

### Detaillierte Beschreibung der Erfindung

Die Chitinosanase gemäß Aspekt (1) der Erfindung ist vorzugsweise ein Enzym, das aus dem *Alternaria alternata* Stamm CCT 2816 der Coleção de Culturas Tropical, Brazil erhältlich ist.

Die bevorzugte Chitinosanase nach Aspekt (2) der Erfindung besetzt eine Aminosäuresequenz, die eines oder beide der Proteinfragmente von SEQ ID NO:1 und 2 umfasst.

Die Sequenzhomologen der Chitinosanase nach Aspekt (2) weisen gemäß der Erfindung eine Ähnlichkeit von wenigstens 80%, vorzugsweise von wenigstens 90%, noch bevorzugter von wenigstens 95% und am bevorzugsten von wenigstens 98% auf. Hierbei sind konservative Austausche von einzelnen oder mehreren zusammenhängenden Aminosäureresten, die Deletion von einzelnen oder mehreren zusammenhängenden Aminosäureresten und die Addition/Einfügung von einzelnen Aminosäureresten oder mehreren zusammenhängenden Aminosäureresten umfasst.

Fragmente von der Chitinosanase von Aspekt (2) oder von den Sequenzhomologen weisen gemäß der Erfindung wenigstens 10 zusammenhängende Aminosäurereste der Ausgangssequenz auf.

Derivate von der Chitinosanase Aspekt (2) bzw. von den Sequenzhomologen oder von den Fragmente derselben umfassen gemäß der Erfindung sowohl Kondensationsprodukte mit anderen funktionellen Protein- oder Peptidstrukturen (z. B. anderen Enzymen, Antikörpern, Sekretionsproteinen, Sequenzen zur Reinigung des Enzyms usw., die direkt oder über einen Linker mit der Chitinosanase verknüpft sein können), als auch mit niedermolekularen organischen Resten (z. B. C- und N-terminalen Resten, Schutzgruppen, Markern usw.) mit Festphasen (z. B. Mikrotiterplatten, Beads usw.).

Die DNA Sequenz gemäß Ausführungsform (3) umfasst sowohl DNA als auch RNA Sequenzen. Ebenfalls umfasst sind Variationen der Sequenzen, die eine Homologie von wenigstens 80%, vorzugsweise wenigstens 90%, besonders bevorzugt wenigstens 98% zu der Ausgangssequenz aufweisen.

Der Vektor gemäß Ausführungsform (4) der Erfindung ist unter anderem ein Transfektionsvektor, der neben der Sequenz noch weiter funktionelle Sequenzen wie Promotoren, Selektionsmarkersequenzen usw. enthalten kann.

Bei der Wirtszelle nach Aspekt (5) der Erfindung, die mit dem Vektor nach Aspekt (4) transformiert/transfiziert ist und/oder die DNA Sequenz nach Aspekt (3) aufweist, handelt es sich um eine Eukaryontenzelle (Pilz, Hefe, Säugerzelle usw.) oder Prokaryontenzelle (*E*. *coli* usw.).

Das Verfahren zur Herstellung einer Chitinosanase nach Aspekt (6) umfasst das Kultivieren einer wie vorstehend definierten Wirtszelle und Isolieren der Chitinosanase aus den kultivierten Wirtszellen und/oder aus der Kulturlösung. Das Verfahren kann weiterhin geeignete Aufreinigungsschritte und/oder Umsetzungen der zunächst erhaltenen Chitinosanase umfassen.

Die Enzymzusammensetzung nach Aspekt (7) der Erfindung kann - in Abhängigkeit mit dem Anwendungsgebiet der Zusammensetzung - nicht nur weitere Enzyme (wie Glukosaminidase) sondern auch Hilfsstoffe wie Stabilisatoren, Puffer usw. enthalten.

Das Verfahren zum Abbau von Chitosan nach Aspekt (8) umfasst die direkte Umsetzung des Chitosans mit einer Chitinosanase nach Aspekt (1) oder (2) der Erfindung oder mit einer Enzymzusammensetzung nach Aspekt (8) der Erfindung. Alternativ kann auch eine Wirtszelle nach Aspekt (5) der Erfindung eingesetzt werden, die die Chitinosanase *in situ* erzeugt. Neben der Chitinosanase der vorliegenden Erfindung können weitere Enzyme eingesetzt werden. Das Verfahren umfasst weiterhin die Aufreinigung der erzeugten Abbauprodukte und den Modifikationen wie nachfolgend beschrieben.

Chitosanabbauprodukte gemäß Aspekt (9) der Erfindung die mittels der erfindungsgemäßen Chitinosanase erzeugt werden, unterscheiden sich von allen oben dargestellten Oligomeren erheblich. Sie sind dadurch charakterisiert, dass sie beliebig viele A- und D-Reste enthalten können, jedoch sind alle D-Reste stets auf der Seite des nicht-reduzierenden Endes, alle A-Reste auf der des reduzierenden Endes konzentriert. Die Formel der Produkte lautet demnach DₙAₘ. Es handelt sich also um partiell acetylierte Chitosanoligomere mit einer blockweisen Verteilung der Acetylreste, mit einem Glukosaminoligomer-Block am nicht-reduzierenden Ende und einem N-Acetyl-Glukosaminoligomer-Block am reduzierenden Ende. Im Gegensatz zu allen anderen größeren Oligomeren ist daher die Architektur jedes Oligomers mit der Bestimmung von n und m eindeutig beschrieben. Diese beiden Werte lassen sich mittels Massenspektroskopie einfach bestimmen.

Daraus folgt auch, dass sich die Produkte des Chitinosanase-Abbaus, im Gegensatz zu denen aller anderen Verfahren, relativ einfach aufreinigen lassen. In einem ersten Schritt können die Oligomere über eine Größenausschluss-Chromatographie nach ihrem Polymerisationsgrad getrennt werden. Wir konnten bereits in der Vergangenheit zeigen, dass sich bei diesem Verfahren selbst vollständig deacetylierte Glukosaminoligomere Dₙ von den um einen Rest kleineren vollständig acetylierten N-Acetyl-Glukosaminoligomeren Aₙ₋₁ sauber trennen lassen. Die einzelnen Oligomergemische lassen sich dann in einem zweiten Schritt über eine Kationenaustausch-Chromatographie nach ihrer Ladungsdichte, und demnach nach der Anzahl der vorhandenen Acetylreste trennen. Im ersten Schritt werden also z.B. die Tetramere DDDA, DDAA und DAAA von den Pentameren DDDDA, DDDAA, DDAAA und DAAAA getrennt, im zweiten Schritt die drei Tetramere bzw. die vier Pentamere jeweils voneinander. Dies ist das erste Verfahren, dass die Produktion solcher partiell acetylierter Chitosanoligomere mit genau bekannter Architektur ermöglicht.

Solche Oligomere könnten einerseits interessante und neue Bioaktivitäten aufweisen, da wir davon ausgehen, dass die biologische Aktivität nicht nur vom Polymerisationsgrad (degree of polymersation, DP) und dem Acetylierungsgrad (degree of acetylation, DA), sondern auch von dem Verteilungsmuster der Acetylreste (pattern of acetylation, PA) abhängt. Zum anderen könnten sie aber auch benutzt werden, um durch Polymerisation partiell acetylierte Chitosanpolymere mit bekanntem Acetylierungsmuster zu erhalten. Diese könnten z.B. so aufgebaut sein, dass sie durch bestimmte körpereigene Enzyme in definierte Produkte abgebaut werden, oder auch durch solche Enzyme nicht abbaubar sind. Es ließe sich also so zum einen die Abbaurate kontrollieren und damit die Verbleibzeit im Körper oder Gewebe, zum anderen könnten biologisch aktive Oligomerprodukte gezielt freigesetzt werden.

Für diese Polymerisation bieten sich prinzipiell zwei mögliche Verfahren an, entweder auf chemischem oder auf enzymatischem Weg. Die chemische Synthese von partiell acetylierten Chitosanoligomeren steckt zwar noch in den Kinderschuhen, ist aber prinzipiell möglich. Der Aufwand ist jedoch schon für die Synthese von Dimeren gewaltig, und er steigt mit der Kettenlänge wahrscheinlich überproportional. Gleichzeitig gehen durch die Vielzahl der benötigten Verfahrensschritte die Ausbeuten stark zurück. Eine Alternative könnte die enzymatische Polymerisation liefern. Allerdings ist kein Enzym bekannt, dass solche Oligomere in der Natur produzieren würde. Die Synthese scheint hier immer über die Polymerisation von N-Acetyl-Glukosamin zu Chitinoligomeren oder Polymeren zu gehen, die dann partiell de-N-acetyliert und gegebenenfalls depolymerisiert werden. Es ist aber bekannt, dass viele chitinolytischen Enzyme eine Glykosyltransferase-Nebenaktivität besitzen. Sie können also nicht nur glykosidische Bindungen spalten, sondern auch Zuckerreste auf andere übertragen. Es bietet sich an, zu versuchen, die hier beschriebene Chitinosanase zu nutzen, um in der Rückreaktion eine Polymerisation zu erreichen. Gegebenenfalls könnte die Rate dieser Rückreaktion durch gezieltes Protein-Engineering verbessert werden. Die Herstellung eines "engineered designer chitosan with inbuilt digestibility in human tissues" kann wie folgt erfolgen:
- Chitosan-Polymer mit mittlerem DA mit Chitinosanase hydrolysieren;
- Produkte über GPC autrennen;
- Dimer- (DA) und Tetramer- (DAAA DDAA DDDA)-Fraktion selektionieren;
- Tetramer-Fraktion über CEC weiter aufreinigen, DAAA selektionieren;
- Dimere und Tetramere über reverse Chitinosanase-Reaktion polymerisieren: DADADADADADADADADADADAAADADADADADADADADAAADADADADADADADADA DADADAAADADADADADADADADAAADADADADADADADADADADADADADADA

### Produkt nach Lysozym-Abbau (in vitro oder in vivo):

| | |
|---|---|
| DADADADADADADADADADADAA | DP 23 |
| ADADADADADADADADAA | DP 18 |
| ADADADADADADADADADADADAA | DP 24 |
| ADADADADADADADADAA | DP 18 |
| ADADADADADADADADADADADADADADA | DP 29 |

Über das Mischungsverhältnis der beiden Oligomere während der Polymerisation kann der mittlere DP der Lysozym-Produkte eingestellt werden, da jedes Tetramer eine Schnittstelle einbaut. Der mittlere DA aller Produkte ist 50%, mit regelmäßigem PA.

Soll der DP auch genau eingestellt werden, so muss die Polymerisation in mehreren Schritten ausgeführt werden:
- Dimer polymerisieren, über GPC z.B. DP 6 (Hexamer) selektionieren: DADADA
- Hexamer und obiges Tetramer im Verhältnis 1/1 polymerisieren, über GPC DP 10 (Dekamer) selektionieren: DADADADAAA und DAAADADADA;
- Dekamer mit Chitinase B von *Serratia marcescens* inkubieren (exo-Chitinase, die vom reduzierenden Ende her AA-Dimere abspaltet): DADADADA, AA und DAAADADADA
- über GPC Dekamer selektionieren: DAAADADADA Dekamer polymerisieren: ...DAAADADADADAAADADADADAAADADADA...

Dieses Polymer wird durch Lysozym in Dekamere gespalten:
...DAA ADADADADAA ADADADADAA ADADADA...

Die besondere Substratspezifität führt auch zur Produktion besonderer Produkte, nämlich partiell acetylierter Chitosanoligomere mit einer genau definierten, blockweisen Verteilung der Acetylreste. Bisher gab es kein Verfahren, mit dem solche Oligomere, die potentiell hochinteressante biologische Aktivitäten aufweisen oder auch als Ausgangssubstanzen für die Synthese neuartiger Polymere mit definierbaren Eigenschaften einsetzbar sind, hergestellt werden könnten.

Die vorliegende Erfindung wird anhand der nachfolgenden Beispiele näher erläutert. Diese schränken die Erfindung jedoch in keiner Weise ein.

### Beispiele

### Beispiel 1: Reinigung und Charakterisierung der Chitinosanase

Anzucht des Pilzes: Die Anzucht von *Alternaria alternata* (Stamm CCT 2816, Coleção de Culturas Tropical, Brazil) als Dauerkultur erfolgte auf mit Agar verfestigtem sterilem MA Medium (Malt Extrakt 2%, Agar 2 %). Die Kultivierung erfolgte zunächst für eine Woche bei 28°C und wechselnden Lichtverhältnissen (12 h hell, 12 h dunkel), die zu einer Sporulation des Pilzes führte. Danach wurden die Platten im Kühlschrank gelagert. Eine Auffrischung erfolgte alle 3 bis 4 Monate. Langfristige Aufbewahrung wurde mit MA-Agar gefülltem Schrägröhrchen, die mit Myzel angeimpft und nach einer kurzen Wachstumsperiode mit sterilem Paraffinöl überschichtet wurden, gewährleistet.

Um eine Vorkultur in Flüssigmedium zu erhalten, wurde MA-Medium ohne Agar mit 2 % Pepton und 2 % Glucose versetzt. Dann wurden zwei bis drei 0,5 cm² große Stücke der *Alternaria-Dauerkultur* in 50 ml des Flüssig-MA-Mediums gegeben und für fünf Tage bei 28 °C in Dunkelheit inkubiert. Mit dieser Vorkultur wurden acht Kolben (je 500 ml) angeimpft und wiederum für 5 bis 7 d bei 28 °C in Dunkelheit inkubiert.

Anreicherung: Die Ernte des Kulturmediums erfolgte durch Zentrifugation bei 13.000 rpm. Der Überstand wurde vorsichtig abgenommen und mittels Vakuumfiltration über einen Membranfilter (Porengröße 0,45 µm) vom restlichen Myzel befreit. Das Volumen des Mediums wurde bestimmt und für die Ultrafiltration eingesetzt. Die Ultrafiltration wurde in einer Ultrafiltrationszelle (Fassungsvolumen 500 ml) mit einem Druck von 3 bar durchgeführt. Die dabei verwendete Membran besaß ein Ausschlussmolekulargewicht von 10 kDa. Das Kulturmedium von *Alternaria alternata* wurde so von ca. 4 I auf 100 ml ankonzentriert. Anschließend wurden die 100 ml in einem Spitzkolben gefriergetrocknet und der trockne Rückstand wurde in 10 ml destilliertem Wasser aufgenommen und für die Gelfiltration eingesetzt. Diese erfolgte mittels PD 10 Säulen. Die PD-10 Säulchen wurden zuerst mit 4x4 ml Na-Acetat-Puffer (50 mM, pH 4,0) äquilibriert und je 2,5 ml des Kulturmediumkonzentrats wurden auf die Säule aufgetragen und nach dem Einsickern mit 3,5 ml Na-Acetat-Puffer eluiert. Mit diesem Eluat wurden anschließend die enzymatischen Messungen und die Proteinbestimmung durchgeführt.

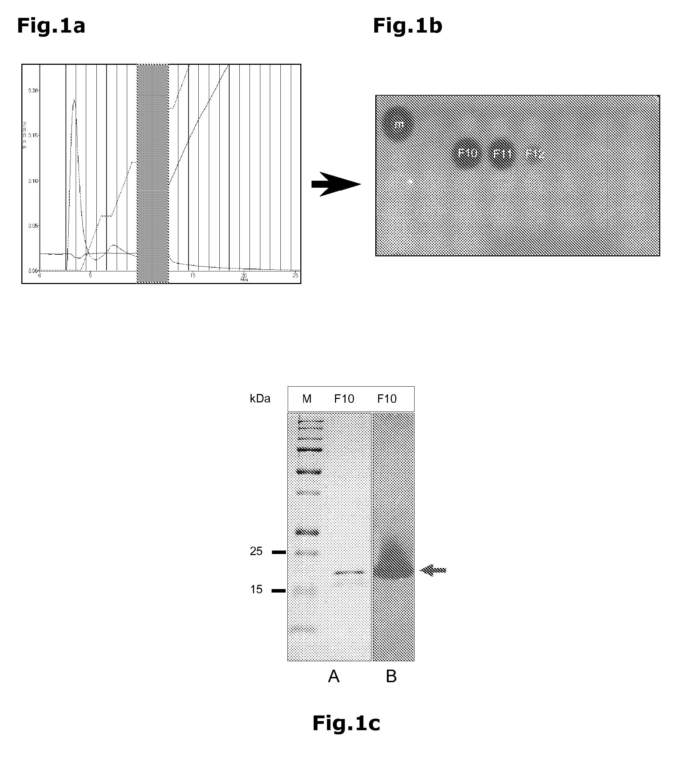

Aufreinigung: Die Aufreinigung der Chitinosanase erfolgte mit Hilfe der FPLC nach dem Prinzip der Kationenaustauschchromatographie. Die Fraktionen wurden mittels eines dot-assays auf chitosanolytische Aktivität getestet, aktive Fraktionen wurden gepoolt und elektrophoretisch auf ihre Reinheit getestet (Fig. 1).

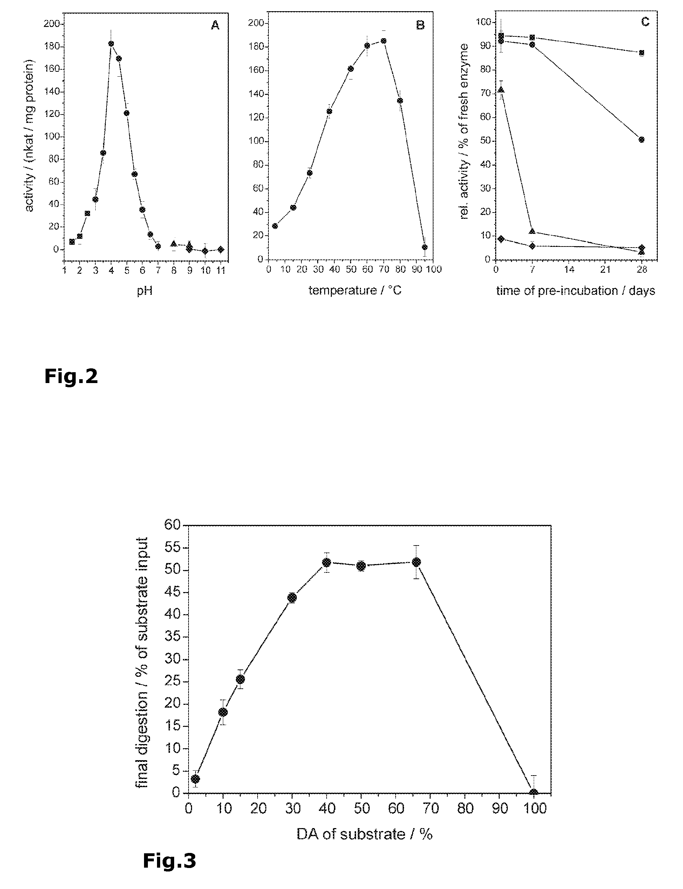

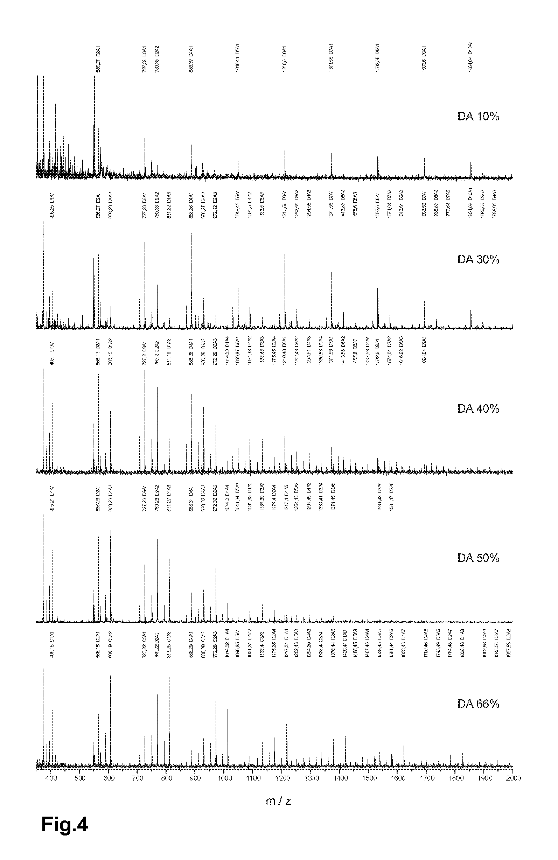

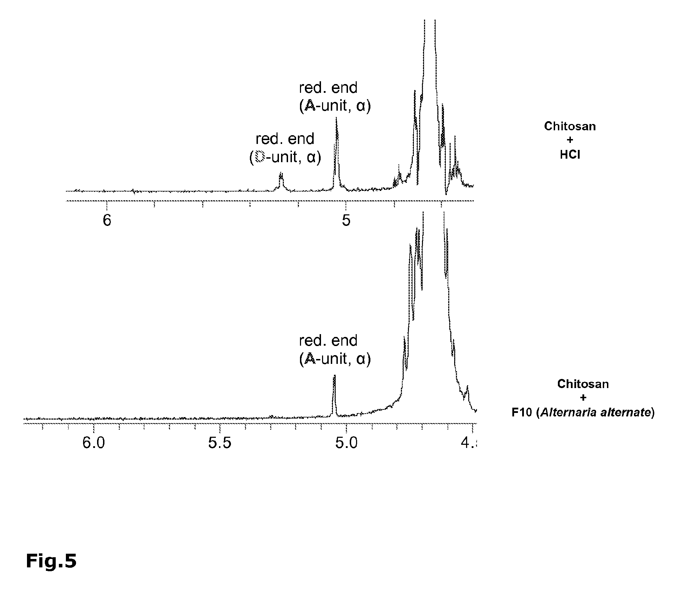

Eigenschaften der Chitinosanase: Das gereinigte Enzym wurde durch die Bestimmung des relativen Molekulargewichts mittels SDS-PAGE (Fig. 1C), des pH-Optimums, des Temperatur-Optimums, der Temperatur-Stabilität (Fig. 2) sowie der Substratspezifität für Chitosane mit unterschiedlichem Acetylierungsgrad (Fig. 3) charakterisiert. Das relative Molekulargewicht betrug 18 kDa. Das pH-Optimum lag bei pH 4 und das Temperatur-Optimum bei 70°C. Nach einer Woche bei 37°C war noch 90 % der Enzymaktivität vorhanden, selbst nach vierwöchiger Lagerung bei dieser Temperatur ließ sich noch die Hälfte der Aktivität nachweisen. Chitosane mit mittlerem Acetylierungsgrad (DA) erwiesen sich als die geeignetsten Substrate. Die Produkte des Chitinosanase-Abbaus verschiedener Substrate wurden mittels Massenspektrometrie (Fig. 4) und NMR (Fig. 5) analysiert. Chitosan mit niedrigem DA lieferten Chitosanoligomere mit nur einem einzigen Acetylrest. Mit zunehmendem DA des Substrats traten vermehrt auch Produkte mit mehreren Acetylresten auf. Alle Produkte trugen eine acetylierte Einheit am reduzierenden Ende.

Vollständig acetylierte Chitin-Oligomere ebenso wie vollständig de-acetylierte Glukosamin-Oligomere wurden auch bei ausgiebiger Inkubation durch die Chitinosanase nicht abgebaut. Das Enzym kann demnach glykosidische Bindungen zwischen zwei acetylierten oder zwischen zwei de-acetylierten Resten nicht hydrolysieren. Da alle Produkte einen acetylierten Rest am reduzierenden Ende tragen, kann man schließen, dass die Chitinosanase die glykosidische Bindung GlcNAc-GlcN, nicht aber die Bindung GlcN-GlcNAc spalten kann.

Die massenspektrometrische Analyse der Produkte bestätigt diese Schlussfolgerung. So treten niemals vollständig acetylierte oder vollständig deacetylierte Produkte auf. Beim Abbau eines Chitosans mit niedrigem DA treten ausschließlich Produkte mit einem einzigen Acetylrest auf. Mit zunehmendem DA des Substrats entstehen auch höher acetylierte Oligomere mit nur einem einzigen deacetylierten Rest. Die vollständig deacetylierten bzw. vollständig acetylierten Bereiche der jeweiligen Produkte werden offenbar nicht weiter abgebaut.

### Beispiel 2: Sequenzierung:

Um die Chitinosanase aus *Alternaria alternata* mit Trypsin zu verdauen, wurde diese wie oben beschrieben bis zu den MonoS-Fraktionen aufgereinigt, anschliessend ankonzentriert und mittels SDS-PAGE aufgetrennt. Um sicher zu gehen, dass es sich bei dem Protein um die Chitinosanase handelte, wurde einem Teil des Geles eine Chitinosanase Aktivitätsfärbung unterzogen. Daraufhin wurde die Chitinosanase-Bande direkt aus dem Gel ausgeschnitten und tryptisch verdaut. Die Peptide wurden mit Acetonitril aus dem Gel extrahiert und mittels LC-MS aufgetrennt bzw. sequenziert. Es wurden folgende Peptidsequenzen identifiziert:
DLNEDLLATPEK (SEQ ID NO:1)
NLKVLLSIGGWSFSANFAGPASSDQK (SEQ ID NO:2)

## Patentansprüche

1. Chitinosanase, die aus dem Pilz *Alternaria alternata* erhältlich ist und die
(a) spezifisch die glykosidische Bindung GlcNAc-GlcN im Chitosan spaltet,
(b) ein mittels SDS-PAGE bestimmtes relatives Molekulargewicht von etwa 18 kDa besitzt
(c) ein pH-Optimum bei pH 4 besitzt und
(d) ein Temperatur-Optimum bei 70°C besitzt.

2. Chitinosanase nach Anspruch 1, das aus dem *Alternaria alternata* Stamm CCT 2816 der Coleção de Culturas Tropical, Brazil erhältlich ist.

3. Chitinosanase, insbesondere nach Anspruch 1 oder 2, die eines oder beide der in SEQ ID NO: 1 und 2 gezeigten Proteinfragmente aufweist.

4. DNA Sequenz, die eine Chitinosanase nach einen der Ansprüche 1 bis 3 codiert.

5. Vektor, der eine DNA Sequenz nach Anspruch 4 enthält.

6. Wirtszelle, die mit dem Vektor nach Anspruch 5 transformiert/transfiziert ist und/oder die DNA Sequenz nach Anspruch 4 aufweist.

7. Verfahren zur Herstellung einer Chitinosanase nach einen der Ansprüche 1 bis 3, umfassend das Kultivieren einer Wirtszelle nach Anspruch 6 und Isolieren der Chitinosanase aus den kultivierten Wirtszellen und/oder aus der Kulturlösung.

8. Enzymzusammensetzung enthaltend eine Chitinosanase nach einen der Ansprüche 1 bis 3.

9. Verfahren zum Abbau von Chitosan, umfassend die Umsetzung des Chitosans mit einer Chitinosanase nach einen der Ansprüche 1 bis 3, mit einer Wirtszelle nach Anspruch 6 oder mit einer Enzymzusammensetzung nach Anspruch 8 und Isolieren der Chitosanabbauprodukte.

10. Chitosanabbauprodukte erhältlich nach dem Verfahren von Anspruch 9.


SEQUENCE LISTING

<110>  Westfaelische Wilhelms-Universitaet Muenster

<120>  Chitinosanase

<130>  072171ep

<160>  2

<170>  PatentIn version 3.3

<210>  1
<211>  26
<212>  PRT
<213>  Alternaria alternata

<400>  1

Asn Leu Lys Val Leu Leu Ser Ile Gly Gly Trp Ser Phe Ser Ala Asn
1               5                   10                  15

Phe Ala Gly Pro Ala Ser Ser Asp Gln Lys
            20                  25

<210>  2
<211>  12
<212>  PRT
<213>  Alternaria alternata

<400>  2

Asp Leu Asn Glu Asp Leu Leu Ala Thr Pro Glu Lys
1               5                   10